# EUROPÄISCHE PATENTSCHRIFT

(11) **EP 2 897 669 B1**
(45) Veröffentlichungstag und Bekanntmachung des Hinweises auf die Patenterteilung: **02.01.2019**
(21) Anmeldenummer: 13765643.5
(22) Anmeldetag: 10.09.2013
(51) Int. Cl.: A61M 1/36, A61M 1/34

(54) **VORRICHTUNG ZUR ERKENNUNG DER REZIRKULATION WÄHREND EINER EXTRAKORPORALEN BLUTBEHANDLUNG**
DEVICE FOR DETECTING RECIRCULATION DURING AN EXTRACORPOREAL BLOOD TREATMENT
DISPOSITIF PERMETTANT D'IDENTIFIER UNE RECIRCULATION PENDANT UN TRAITEMENT SANGUIN EXTRACORPOREL

(30) Priorität: 21.09.2012 DE 102012018628; 21.09.2012 US 201261703845 P
(43) Veröffentlichungstag der Anmeldung: 29.07.2015
(73) Patentinhaber: Fresenius Medical Care Deutschland GmbH, 61352 Bad Homburg v.d.H. (DE)
(72) Erfinder: NÜRNBERGER, Thomas, 97705 Burkardroth (DE)
(74) Vertreter: Oppermann, Frank
(86) Internationale Anmeldenummer: PCT/EP2013/002718
(87) Internationale Veröffentlichungsnummer: WO 2014/044365

(56) Entgegenhaltungen:
- EP-A1- 1 595 560
- DE-A1-102008 003 714
- US-A- 6 117 099

## Beschreibung

Die Erfindung betrifft eine Vorrichtung zur Erkennung der Rezirkulation für eine extrakorporale Blutbehandlungsvorrichtung sowie eine extrakorporale Blutbehandlungsvorrichtung mit einer Vorrichtung zur Erkennung der Rezirkulation.

Zur Entfernung von harnpflichtigen Substanzen und zum Flüssigkeitsentzug werden verschiedene Verfahren zur extrakorporalen Blutbehandlung bzw. -reinigung eingesetzt. Bei der Hämodialyse wird das Blut des Patienten außerhalb des Körpers in einem Dialysator gereinigt. Der Dialysator weist eine Blutkammer und eine Dialysierflüssigkeitskammer auf, die durch eine semipermeable Membran getrennt sind. Während der Behandlung strömt Blut des Patienten durch die Blutkammer. Um das Blut effektiv von harnpflichtigen Substanzen zu reinigen, wird die Dialysierflüssigkeitskammer kontinuierlich von frischer Dialysierflüssigkeit durchströmt.

Während bei der Hämodialyse (HD) der Transport der kleineren molekularen Substanzen durch die Membran des Dialysators im Wesentlichen durch die Konzentrationsunterschiede (Diffusion) zwischen der Dialysierflüssigkeit und dem Blut bestimmt wird, werden bei der Hämofiltration (HF) im Plasmawasser gelöste Substanzen, insbesondere höhermolekulare Stoffe, durch einen hohen Flüssigkeitsstrom (Konvektion) durch die Membran des Dialysators effektiv entfernt. Bei der Hämofiltration fungiert der Dialysator als Filter. Eine Kombination aus beiden Verfahren ist die Hämodialfiltration (HDF).

Bei der Hämo(dia)filtration wird ein Teil der dem Blut durch die Membran des Dialysators entzogenen Flüssigkeit durch eine sterile Substitutionsflüssigkeit ersetzt, die im Allgemeinen entweder stromauf des Dialysators oder stromab des Dialysators dem extrakorporalen Blutkreislauf zugeführt wird. Die Zufuhr der Substitutionsflüssigkeit

Bei der Hämo(dia)filtration wird ein Teil der dem Blut durch die Membran des Dialysators entzogenen Flüssigkeit durch eine sterile Substitutionsflüssigkeit ersetzt, die im Allgemeinen entweder stromauf des Dialysators oder stromab des Dialysators dem extrakorporalen Blutkreislauf zugeführt wird. Die Zufuhr der Substitutionsflüssigkeit stromauf des Dialysators wird auch als Prädilution und die Zufuhr stromab des Dialysators als Postdilution bezeichnet.

Es sind Vorrichtungen zur Hämo(dia)filtration bekannt, bei denen die Dialysierflüssigkeit online aus Frischwasser und Dialysierflüssigkeitskonzentrat und die Substitutionsflüssigkeit online aus der Dialysierflüssigkeit hergestellt werden.

Bei den bekannten Hämo(dia)filtrationsvorrichtungen wird die Substitutionsflüssigkeit (Substituat) dem extrakorporalen Blutkreislauf von dem Flüssigkeitssystem der Maschine über eine Substituatzuführleitung zugeführt. Bei der Prädilution führt die Substituatleitung zu einer Anschlussstelle an der arteriellen Blutleitung stromauf des Dialysators oder Filters, während bei der Postdilution die Substituatleitung zu einer Anschlussstelle an der venösen Blutleitung stromab des Dialysators oder Filters führt.

Bei den bekannten Verfahren der chronischen Blutreinigungstherapie, wie Hämodialyse, Hämofiltration und Hämodiafiltration, wird im Allgemeinen als Zugang zum Blutgefäßsystem des Patienten operativ eine Gefäßverbindung (shunt) zwischen einer Arterie und einer Vene angelegt. Wenn nachfolgend von einer "Fistel" die Rede ist, wird darunter jede Art der Verbindung zwischen einer Vene und einer Arterie des Patienten verstanden.

Das durch die Fistel fließende Blut wird nur während der eigentlichen Dialysebehandlung benutzt. Im dialysefreien Zeitraum entspricht der Blutfluss in der Fistel einem funktionellen Links/Rechts-Shunt, bei dem ein Anteil des arteriellen Blutes aus dem Herzminutenvolumen (HMV) unter Umgehung einer peripheren Nutzung direkt dem venösen System und dem Herzen zugeführt wird. Der Fistelfluss rezirkuliert über Herz und Lungen. Der fraktionelle Anteil des Fistelflusses am Herzminutenvolumen wird als kardiopulmonare Rezirkulation definiert.

Die kardiopulmonare Rezirkulation hat nicht nur Auswirkungen auf die Kreislaufbelastung des Patienten, sondern auch auf die Effizienz der Dialyse. Da das dialysierte Blut aus dem extrakorporalen Kreislauf unter Umgehung der systemischen Kreislaufgebiete dem venösen Rückstrom aus dem großen Körperkreislauf beigemischt wird, kommt es zu einer systematischen Reduktion in der Konzentration dialysierbarer Bestandteile im arteriellen Blut (D. Schneditz et al.: Cardiopulmonary recirculation during hemodialysis. Kidney Int. 42: 1450- 1456, 1992).

Für die Funktionsfähigkeit der Fistel ist deren Perfusion von Bedeutung. Sinkt der Fistelfluss unter einen kritischen Wert, dann steigt das Risiko einer Fistelthrombose mit dem möglichen Verlust des Gefäßzuganges, was in der Dialysebehandlung eine erhebliche Komplikation darstellt (W. Bay et al.: Color Doppler flow predicts PTFE graft failure, J. Am. Soc. Nephrol. 5: 407 (1994)). Ist der Fistelfluss während der Dialysebehandlung kleiner als der extrakorporale Blutfluss (Q_{B}), kommt es zur lokalen Fistelrezirkulation, wobei eine Fraktion des dialysierten und mit der venösen Blutleitung zur Fistel zurückgeführten Bluts über die arterielle Blutleitung dem Dialysator wieder zugeführt wird. Die Fistelrezirkulation R_{A} bewirkt eine bedeutende Verminderung der Dialyseeffizienz (F. Gotch: "Models to predict recirculation and its effect on treatment time in single-needle dialysis" First Intl. Symposium on Single-Needle Dialysis, Hrsg.: S. Rignoir. R. Vanholder und P. Invanovich, Cleveland, ISAO Press, 1984, Seite 305 ff.). Die Messung der Qualität des Gefäßzuganges ist somit ein wichtiges Mittel zur Qualitätssicherung bei der Dialysebehandlung.

Die WO 98/32477 beschreibt ein Verfahren zur Messung der Rezirkulation R, d.h. der Summe der Fistelrezirkulation (R_{A}) und der kardiopulmonalen Rezirkulation R_{CP}. Bei dem bekannten Verfahren wird eine physikalische oder chemische Kenngröße der Dialysierflüssigkeit im Dialysierflüssigkeitsweg stromauf des Dialysators verändert, die zu einer Änderung der physikalischen oder chemischen Kenngröße auf der Blutseite führt. Die Änderung der Kenngröße auf der Blutseite führt zu einer Änderung der Kenngröße der Dialysierflüssigkeit stromab der Dialysierflüssigkeitskammer des Dialysators. Zur Bestimmung der Rezirkulation wird die Kenngröße im Dialysierflüssigkeitsweg stromab gemessen werden.

Die US 5 830 365 beschreibt ein Verfahren zur Bestimmung der kardiopulmonalen Rezirkulation, das auf zwei kurz aufeinander folgenden Messungen der Rezirkulationsfraktion beruht, die automatisch vor und nach der Umkehrung des Blutflusses durchgeführt werden. Nachteilig ist, dass das bekannte Verfahren die Umkehr des Blutflusses erfordert.

Aus der EP 1 595 560 A1 ist ein Verfahren zur Bestimmung der Rezirkulation bekannt, bei dem durch Einschalten und/oder Ausschalten der Substituatpumpe die Viskosität des Bluts in der arteriellen oder venösen Blutleitung verändert wird. Die Veränderung der Viskosität des Bluts führt zu einem Druckabfall oder Druckanstieg auf der venösen oder arteriellen Seite des extrakorporalen Blutkreislaufs, der erfasst wird, um auf der Grundlage der Veränderung des Drucks eine Rezirkulation zu erkennen. Das bekannte Verfahren sieht eine Veränderung der Substituatrate durch Einschalten und/oder Ausschalten der Substituatpumpe vor, während die Blutpumpe im extrakorporalen Blutkreislauf weiterhin Blut mit der vorgegebenen Blutflussrate fördert.

Der Erfindung liegt die Aufgabe zugrunde, ein Vorrichtung zu schaffen, mit der eine Rezirkulation während einer extrakorporalen Blutbehandlung schnell und zuverlässig erkannt werden kann, so dass sich die Überprüfung des Zustandes der Fistel während der extrakorporalen Blutbehandlung routinemäßig ohne großen Aufwand durchführen lässt.

Die Lösung dieser Aufgabe erfolgt erfindungsgemäß mit den Merkmalen des Patentanspruchs 1. Die Gegenstände der abhängigen Ansprüche betreffen vorteilhafte Ausführungsformen der Erfindung.

Die erfindungsgemäße Vorrichtung beruht auf einer gezielten Hämodilution durch die Gabe eines Bolus einer Substitutionsflüssigkeit (Substituat). Die Hämodilution führt infolge einer Veränderung der Viskosität der im extrakorporalen Kreislauf strömenden Flüssigkeit zu einer Veränderung der Druckverhältnisse im venösen und arteriellen Zweig des extrakorporalen Blutkreislaufs. Eine Rezirkulation wird auf der Grundlage der Erfassung der Druckänderung im extrakorporalen Kreislauf erkannt. Die Druckänderung kann sich dabei als eine Erhöhung des Drucks in der arteriellen Blutleitung oder eine Verringerung des Drucks in der venösen Blutleitung zeigen.

Die erfindungsgemäße Vorrichtung zeichnet sich durch eine gezielte Reduzierung der Blutflussrate während der Gabe des Bolus von Substituat aus. Während der extrakorporalen Blutbehandlung wird die Blutflussrate innerhalb eines vorgegeben Zeitintervalls von einer vorgegebenen aktuellen Blutflussrate, die nachfolgend als erste Blutflussrate bezeichnet wird, auf eine Blutflussrate reduziert, die nachfolgend als zweite Blutflussrate bezeichnet wird. Vorzugsweise entspricht die zweite Blutflussrate einer Mindestblutflussrate, die vom Arzt vorgegeben werden kann. Die Reduzierung der Blutflussrate wird erfindungsgemäß zumindest teilweise durch die Bolusgabe von Substituat wieder kompensiert. Vorzugsweise wird das reduzierte Volumen an Blut in dem vorgegebenen Zeitintervall vollständig durch Substituat ersetzt. Die Bolusgabe sollte zeitgleich mit der Reduzierung der Blutflussrate einhergehen, d.h. das vorgegebene Zeitintervall, in dem die Blutbehandlung mit einer reduzierten Blutflussrate erfolgt, sollte dem Zeitintervall entsprechen, in dem der Substitut-Bolus gegeben wird. Es können aber auch gewisse zeitliche Verschiebungen zwischen den Zeitintervallen der Reduzierung der Blutflussrate und der Bolusgabe gegeben sein.

Es hat sich gezeigt, dass mit der gleichzeitigen Bolusgabe von Substituat und der Reduzierung der Blutflussrate die Zusammensetzung der durch den extrakorporalen Kreislauf strömenden Flüssigkeit, d. h. des mit Substituat verdünnten Bluts, für die Erkennung einer Rezirkulation optimiert wird. Aus der gezielten Veränderung der Blutfluss- und Substituatrate innerhalb des Zeitintervalls ergeben sich größere Druckänderungen im venösen und arteriellen Zweig des extrakorporalen Kreislaufs, die sich zur Erkennung einer Rezirkulation mit höherer Sicherheit erfassen lassen. Dadurch wird die Empfindlichkeit und Zuverlässigkeit des Messverfahrens insgesamt verbessert.

Die erfindungsgemäße Vorrichtung zur Erkennung der Rezirkulation kann eine separate Baugruppe bilden oder Bestandteil der extrakorporalen Blutbehandlungsvorrichtung sein. sich zur Erkennung einer Rezirkulation mit höherer Sicherheit erfassen lassen. Dadurch wird die Empfindlichkeit und Zuverlässigkeit des Messverfahrens insgesamt verbessert. Die erfmdungsgemäße Vorrichtung zur Erkennung der Rezirkulation kann eine separate Baugruppe bilden oder Bestandteil der extrakorporalen Blutbehandlungsvorrichtung sein. Vorzugsweise ist die erfindungsgemäße Vorrichtung zur Erkennung der Rezirkulation Bestandteil der Blutbehandlungsvorrichtung, die bereits über die wesentlichen Komponenten zur Durchführung des erfindungsgemäßen Verfahrens verfügt.

Die Vorrichtung zur Bestimmung der Rezirkulation weist eine Messeinheit zum Messen des Drucks im extrakorporalen Kreislauf in der arteriellen und/oder venösen Blutleitung und eine Auswerteinheit zur Erkennung der Rezirkulation auf der Grundlage der Druckmessung auf. Darüber hinaus verfügt die Vorrichtung zur Erkennung der Rezirkulation über eine Steuereinheit, die mit der Einrichtung zum Fördern von Blut der Blutbehandlungsvorrichtung und der Einrichtung zum Zuführen von Substituat der Blutbehandlungsvorrichtung zusammenwirkt. Die Steuereinheit sieht einen Betriebsmodus zur Erkennung der Rezirkulation vor, in dem die Einrichtung zum Fördern von Blut derart angesteuert wird, dass in einem vorgegebenen Zeitintervall die vorgegebene erste Blutflussrate Q_{b1} auf die zweite Blutflussrate Q_{b2} reduziert wird, und die Einrichtung zum Zuführen von Substituat derart angesteuert wird, dass in dem vorgegebenen Zeitintervall Substituat mit einer vorgegebenen Substituatrate Qₛ dem Blut in der arteriellen Blutleitung stromauf des Dialysators oder Filters zugeführt wird. Innerhalb des vorgegeben Zeitintervalls können die Förderraten konstant sein oder ein bestimmtes Profil haben, beispielsweise kontinuierlich oder diskontinuierlich ansteigen und abfallen.

Bei einer bevorzugten Ausführungsform ist die Steuereinheit derart ausgebildet ist, dass die Einrichtung zum Zuführen von Substituat in dem vorgegebenen Zeitintervall in dem Betriebsmodus zur Erkennung der Rezirkulation derart angesteuert wird, dass die Differenz zwischen der vorgegebenen Substituatrate Qₛ und der Differenz zwischen der ersten Blutflussrate Q_{b1} und der zweiten Blutflussrate Q_{b2} einem bestimmten Wert entspricht. Dadurch wird die Reduzierung der Blutflussrate zumindest teilweise mit der Zufuhr von Substituat kompensiert. Vorzugsweise ist der bestimmte Wert aber null, so dass ein vollständiger Ausgleich erfolgt.

Eine besonders bevorzugte Ausführungsform sieht vor, dass die Auswerteinheit bei der Erkennung einer Druckänderung ein das Vorliegen einer Rezirkulation signalisierendes Steuersignal erzeugt, wobei die Vorrichtung zur Bestimmung der Rezirkulation eine das Steuersignal der Auswerteinheit empfangende optische und/oder akustische Signaleinheit aufweist, die ein akustisches und/oder optisches Signal erzeugt, wenn die Signaleinheit von der Auswerteinheit das Steuersignal empfängt.

Aufgrund der Hämodilution infolge der Bolusgabe mit der Reduzierung des Blutflusses ergibt sich zunächst ein Druckabfall im venösen Zweig des extrakorporalen Blutkreislaufs. Wenn eine Rezirkulation vorliegt, gelangt ein Teil des Blutvolumens direkt in den arteriellen Zweig des Blutkreislaufs, die zu einem zeitlich verschobenen Druckanstieg im arteriellen Zweig des Blutkreislaufs führt, da sich infolge der Verringerung der Viskosität der Druckabfall an der arteriellen Kanüle reduziert. Folglich kann die Rezirkulation durch einen Druckanstieg im arteriellen Zweig des Blutkreislaufs erkannt werden.

Bei einer besonders bevorzugten Ausführungsform ist die Auswerteinheit derart ausgebildet ist, dass der Betrag der Änderung des Drucks in der arteriellen oder venösen Blutleitung mit einem vorgegebenen Grenzwert verglichen wird, wobei die Auswerteinheit ein das Vorliegen einer Rezirkulation anzeigendes Steuersignal erzeugt, wenn der Betrag der Druckänderung größer als der vorgegebene Grenzwert ist. Zur Erkennung einer Rezirkulation können aber auch andere Auswertverfahren angewandt werden. Allein entscheidend ist, dass der auf die Rezirkulation zurückzuführende Druckanstieg erkannt wird.

Im Allgemeinen ist es ausreichend, wenn eine Rezirkualtion erkannt wird, um eine Aussage über den Zustand des Gefäßzugangs machen zu können. Die erfindungsgemäße Vorrichtung zur Erkennung der Rezirkulation lässt aber auch eine Bestimmung der Rezirkulation zu. Zur Berechnung des prozentualen Anteils der Rezirkulation ist die Auswerteinheit vorzugsweise derart ausgebildet, dass das Verhältnis zwischen dem Betrag der Änderung des in der arteriellen Blutleitung gemessenen Drucks und des in der venösen Blutleitung gemessenen Drucks berechnet wird. Vorzugsweise werden die Integrale der mit den Druckmesseinheiten gemessenen Drucksignale zueinander ins Verhältnis gesetzt.

Die Rezirkulation kann auch mit den anderen im Stand der Technik bekannten Verfahren genau bestimmt werden, sobald die erfindungsgemäße Vorrichtung eine Rezirkulation erkannt hat.

Zur schnellen Einleitung einer Rezirkulationsprüfung sieht eine weitere besonders bevorzugte Ausführungsform eine Betätigungseinheit für die Steuereinheit vor, die derart ausgebildet ist, dass nach Betätigung eines Betätigungsorgans die Steuereinheit den Betriebsmodus zur Erkennung der Rezirkulation vorgibt, so dass die hierzu erforderlichen Prozessschritte automatisch durchgeführt werden. Damit kann die Rezirkulationsprüfung schnell eingeleitet und dann automatische durchgeführt werden. Die Betätigungseinheit kann ein mechanischer Schalter oder Taster oder ein Bedienfeld eines Touch-Screens sein.

Nachfolgend wird ein Ausführungsbeispiel der Erfindung unter Bezugnahme auf die Zeichnungen im Einzelnen erläutert.

Es zeigen:
- Fig. 1: eine extrakorporale Blutbehandlungsvorrichtung mit einer Vorrichtung zur Erkennung der Rezirkulation in stark vereinfachter schematischer Darstellung,
- Fig. 2: ein Diagramm zur Veranschaulichung der Flussraten im extrakorporalen Blutkreislauf während der Bolusgabe,
- Fig. 3: ein erstes Ausführungsbeispiel des zeitlichen Verlaufs des Drucks im arteriellen und venösen Zweig des extrakorporalen Blutkreislaufs während der Bolusgabe bei einer Rezirkulation,
- Fig. 4: ein zweites Ausführungsbeispiel des zeitlichen Verlaufs des Drucks im arteriellen und venösen Zweig des extrakorporalen Blutkreislaufs während der Bolusgabe bei einer Rezirkulation und
- Fig. 5: den zeitlichen Verlauf des Drucks im arteriellen und venösen Zweig des extrakorporalen Blutkreislaufs während der Bolusgabe, wenn keine Rezirkulation erfolgt.

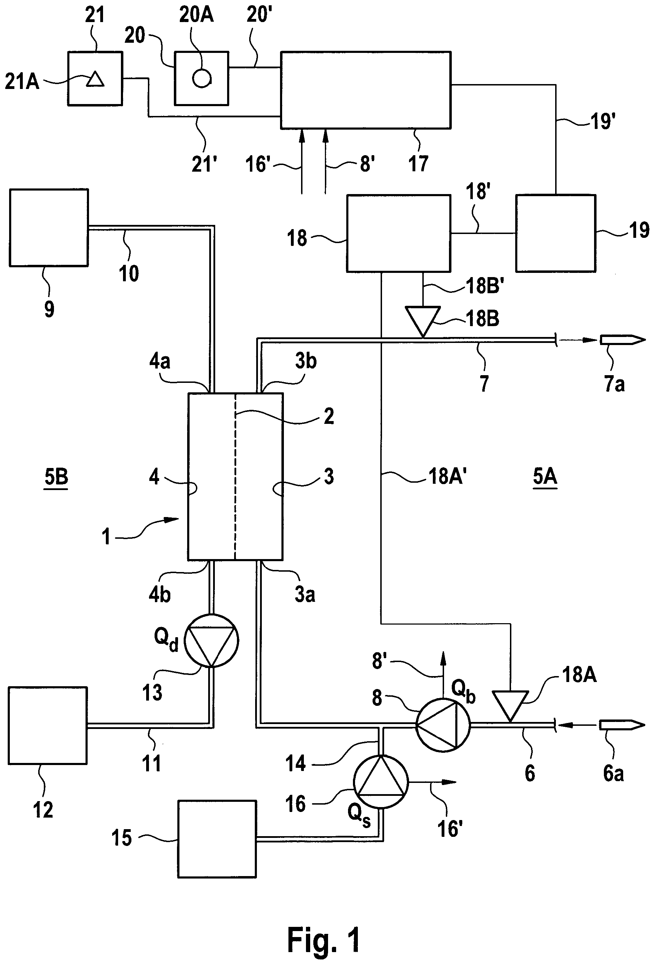

Fig. 1 zeigt in vereinfachter schematischer Darstellung nur die wesentlichen Komponenten einer extrakorporalen Blutbehandlungsvorrichtung, die über eine Vorrichtung zur Erkennung der Rezirkulation verfügt.

Bei der vorliegenden Blutbehandlungsvorrichtung handelt es sich um eine Hämo(dia)filtrationsvorrichtung, die einen Dialysator 1 aufweist, der durch eine semipermeable Membran 2 in eine von Blut durchflossene erste Kammer 3, die nachfolgend als Blutkammer bezeichnet wird, und eine von Dialysierflüssigkeit durchflossene zweite Kammer 4 getrennt ist, die als Dialysierflüssigkeitskammer bezeichnet wird. Die erste Kammer 3 ist in einen extrakorporalen Blutkreislauf 5A geschaltet, während die zweite Kammer 4 in das Dialysierflüssigkeitssystem 5B der Hämo(dia)filtrationsvorrichtung geschaltet ist.

Der extrakorporale Blutkreislauf 5A umfasst eine arterielle Blutleitung 6, die von einer arteriellen Kanüle 6a zu dem Einlass 3a der Blutkammer 3 führt, und eine venöse Blutleitung 7, die von dem Auslass 3b der Blutkammer 3 des Dialysators 1 abgeht und zu einer venösen Kanüle 7a führt. Die arterielle und venöse Kanüle sind an einer nicht dargestellten Fistel (shunt) des Patienten angeschlossen. Das Blut des Patienten wird durch die Blutkammer 3 des Dialysators 1 mit einer arteriellen Blutpumpe 8 gefördert, die an der arteriellen Blutleitung 6 angeordnet ist. Die Blutpumpe 8 führt der Blutkammer 3 des Dialysators Blut mit einer vorgebebenen Blutflussrate Q_{b} zu, die als erste Blutflussrate bezeichnet wird. Die erste Blutflussrate kann während der Blutbehandlung verändert werden. Die Blutleitungen 6, 7 und der Dialysator 3 bilden ein zur einmaligen Verwendung bestimmtes Disposable, das für die Dialysebehandlung in die Dialysevorrichtung eingelegt wird. Zur Eliminierung von Luftblasen können in die arterielle und venöse Blutleitung Luftabscheider (Tropfkammer) geschaltet sein.

Die frische Dialysierflüssigkeit wird in einer Dialysierflüssigkeitsquelle 9 bereitgestellt. Von der Dialysierflüssigkeitsquelle 9 führt eine Dialysierflüssigkeitszuführleitung 10 zu dem Einlass 4a der Dialysierflüssigkeitskammer 4 des Dialysators 1. Von dem Auslass 4b der Dialysierflüssigkeitskammer 4 führt eine Dialysierflüssigkeitsabführleitung 11 zu einem Abfluss 12. In die Dialysierflüssigkeitsabführleitung 11 ist eine Dialysierflüssigkeitspumpe 13 geschaltet.

Während der Dialysebehandlung kann Substitutionsflüssigkeit (Substituat) über eine Substituatleitung 14 dem extrakorporalen Blutkreislauf 5A aus einer Substituatquelle 15 zugeführt werden. Die Substituatleitung 14 ist an einen Leitungsabschnitt der arteriellen Blutleitung 6 stromab der Blutpumpe 8 und stromauf der Blutkammer 3 des Dialysators 1 angeschlossen. Das Substituat wird mit einer Substituatpumpe 16 gefördert, die der arteriellen Blutleitung 6 Substituat mit einer vorgegebenen Substituatrate Qₛ zuführt. Das Substitut kann eine aus der Diaylsierflüssigkeit während der Behandlung hergestellte Flüssigkeit sein. Das Substituat kann aber auch eine in der Substituatquelle bereitgestellte Flüssigkeit, beispielsweise eine Kochsalzlösung sein.

Die Vorrichtung zur Erkennung einer Rezirkulation verfügt über eine Steuereinheit 17, die Bestandteil der zentralen Steuer- und Recheneinheit der Blutbehandlungsvorrichtung sein kann. Die Blutpumpe 8 und die Substituatpumpe 16 sind über Steuerleitungen 8', 16' mit der Steuereinheit 17 der Vorrichtung zur Erkennung der Rezirkulation verbunden. Die Steuereinheit schaltet die Pumpen 8, 16 ein- und aus und gibt für die Pumpen bestimmte Flussraten vor.

Darüber hinaus verfügt die Vorrichtung zur Erkennung der Rezirkulation über eine Messeinheit 18 mit einem arteriellen Drucksensor 18A zum Messen des Drucks in der arteriellen Blutleitung 6 stromauf der Blutpumpe 8 und einem venösen Drucksensor 18B zum Messen des Drucks in der venösen Blutleitung 7 stromab der Blutkammer des Dialysators. Der arterielle und venöse Drucksensor 18A, 18B sind über Datenleitungen 18A', 18B' mit der Messeinheit 18, und die Messeinheit 18 ist mit einer Datenleitung 18' mit einer Auswerteinheit 19 der Vorrichtung zur Erkennung einer Rezirkulation verbunden, die mit einer Datenleitung 19' wieder mit der Steuereinheit 17 verbunden ist.

Des Weiteren verfügt die Vorrichtung zur Erkennung der Rezirkulation über eine Betätigungseinheit 20 mit einem Betätigungsorgan 20A, beispielsweise ein Taster, und eine Signaleinheit 21, die einen optischen und/oder akustischen Signalgeber 21A aufweist. Die Betätigungseinheit 20 und Signaleinheit 21, die ebenfalls Bestandteil der Blutbehandlungsvorrichtung sein können, sind über Steuer- oder Datenleitungen 20', 21' mit der Steuereinheit 17 verbunden.

Die Steuereinheit 17 kann eine Datenverarbeitungseinheit (Mikroprozessor) sein, auf der ein Datenverarbeitungsprogramm läuft, um die für die Durchführung des erfindungsgemäßen Verfahrens erforderlichen Schritte auszuführen. Die Funktionsweise der Steuereinheit wird nachfolgend im Einzelnen beschrieben. Die Steuereinheit ist derart konfiguriert (programmiert), dass die einzelnen Komponenten der Blutbehandlungsvorrichtung wie folgt angesteuert werden.

Während der Blutbehandlung fördert die Blutpumpe 8 das Blut mit einer vorgegebenen ersten Blutflussrate Q_{b1}, die während der Blutbehandlung variieren kann. Zur Einleitung der Rezirkulationsprüfung wird das Betätigungsorgan 20A betätigt, beispielsweise der Taster gedrückt. Nach Drücken des Tasters gibt die Steuereinheit 17 den Betriebsmodus zur Erkennung der Rezirkulation vor, so dass die Blutpumpe 8 und die Substituatpumpe 16 zur Gabe eines Substituatbolus bei reduziertem Blutfluss angesteuert werden. Die Steuereinheit 17 ist derart konfiguriert (programmiert), dass die Blutpumpe 8 das Blut innerhalb eines vorgegebenen Zeitintervalls ΔT mit einer reduzierten zweiten Blutflussrate Q_{b2} fördert. Die Steuereinheit 17 berechnet die Differenz von der ersten Blutflussrate Q_{b1} und der zweiten Blutflussrate Q_{b2} und gibt für die Substituatrate Qₛ eine Rate vor, die der Differenz von der ersten und der zweiten Blutflussrate Q_{b1}, Q_{b2} entspricht.

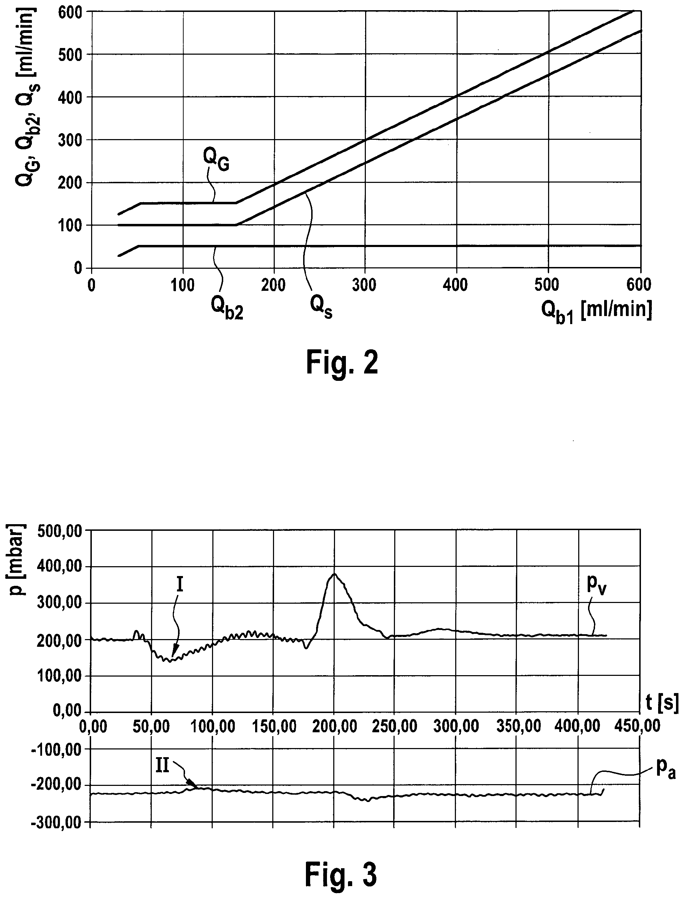

Fig. 2 veranschaulicht die Vorgabe der Substituatrate Qₛ während der Bolusgabe für unterschiedliche Flussraten. Die Blutflussrate wird von einem Wert Q_{b1} auf einen minimalen Wert Q_{b2}= Qₘᵢₙ, beispielsweise 50 ml/min, reduziert. Für eine Blutflussrate Q_{b1} vor der Bolusgabe von 400 ml/min ergibt sich beispielsweise für die Substituatrate Qₛ während der Bolusgabe ein Wert von 350 ml/min, woraus sich als Summe Q_{G} = Qₛ + Q_{b2} wieder der Wert von 400 ml ergibt.

In dem Betriebsmodus zur Rezirkulationserkennung misst der arterielle und venöse Drucksensor 18A, 18B der Messeinheit 18 den Druck in der arteriellen und venösen Blutleitung 6, 7, wobei die Auswerteinheit 19 die Drucksignale auswertet.

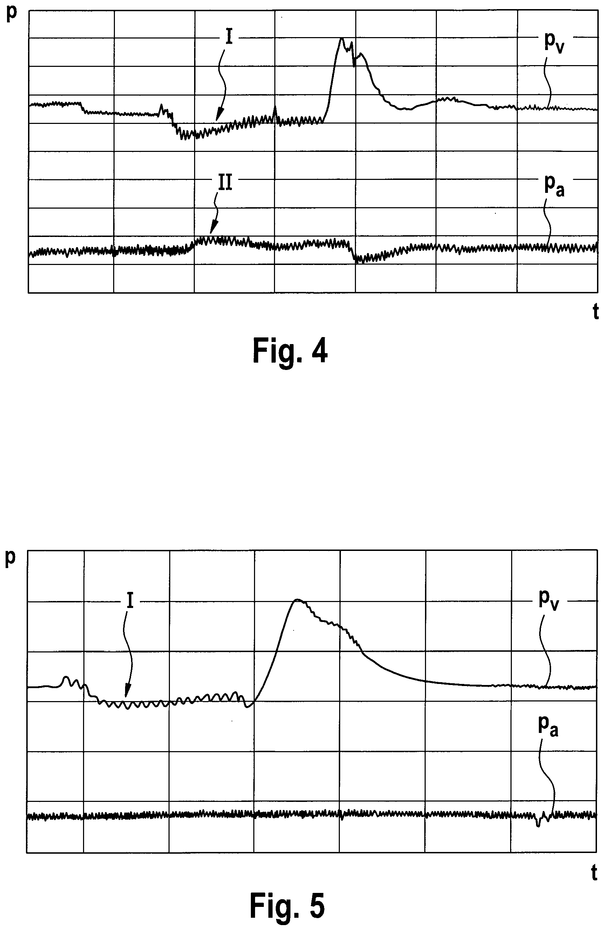

Figur 3 zeigt den gemessenen zeitlichen Verlauf des venösen und arteriellen Drucks pᵥ (mbar) und pₐ (mbar). Nach dem Substituatbolus bei reduziertem Blutfluss ist aufgrund der Hämodilution ein Druckabfall I um einen bestimmten Betrag in der venösen Blutleitung 7 und ein Druckanstieg II in der arteriellen Blutleitung 6 zu erkennen, die mit den Bezugszeichen I und II gekennzeichnet sind. Bei diesem Ausführungsbeispiel beträgt die Rezirkulation 20 %. Zum Vergleich zeigt Fig. 4 den zeitlichen Verlauf des venösen und arteriellen Drucks pᵥ (mbar) und pₐ (mbar) bei einer Rezirkulation von 40%, während Fig. 5 den zeitlichen Verlauf des venösen und arteriellen Drucks pᵥ (mbar) und pₐ (mbar) zeigt, wenn keine Rezirkulation vorliegt. Deutlich ist zu erkennen, dass nur für den Fall der Rezirkulation ein arterieller Druckanstieg zu verzeichnen ist.

Der in den Figuren 3 bis 5 gezeigte charakteristische Verlauf des Drucksignals ist auf die veränderte Viskosität des Blutes zurückzuführen. Aufgrund der Bolusgabe wird die Viskosität des Blutes in der venösen Blutleitung 7 stromab der Blutkammer wegen der Zufuhr von Substituat verringert. Folglich tritt an der venösen Kanüle 7a ein geringerer Druckabfall auf. Dadurch fällt der venöse Druck ab. Daraufhin erhöht sich die Viskosität des Blutes in der venösen Blutleitung 7 wieder, so dass der venöse Druck wieder ansteigt. Aufgrund der Fistelrezirkulaltion gelangt ein Teil des verdünnten Bluts über die Fistel direkt in die arterielle Blutleitung 6, was zu einer Viskositätsverringerung führt, weshalb der Druck (Unterdruck) in der arteriellen Bluteitung ansteigt. Aus dem arteriellen Druckanstieg oder venösen Druckabfall schließt die Auswerteinheit 19 auf das Vorliegen einer Fistelrezirkuation. Hierzu bestimmt die Auswerteinheit 19 den Betrag des Druckanstiegs in der arteriellen Blutleitung 6 und vergleicht den Betrag mit einem vorgegebenen Grenzwert. Alternativ kann die Auswerteinheit 19 auch den Betrag des Druckabfalls in der venösen Blutleitung 7 bestimmen und den Betrag mit einem vorgegebenen Grenzwert vergleichen. Wenn der Grenzwert bei einer signifikanten Änderung des Drucks überschritten wird, erzeugt die Auswerteinheit 19 ein Steuersignal, das eine Rezirkulation signalisiert. Die Signaleinheit 21 empfängt das Steuersignal der Auswerteinheit 19 über die Steuereinheit 17, so dass der Signalgeber 21A einen akustischen und/oder optischen Alarm erzeugt. Der behandelnde Arzt kann dann die erforderlichen Maßnahmen ergreifen.

Die erfindungsgemäße Vorrichtung erlaubt auch die Bestimmung der Rezirkulation. Als Maß für die Rezirkulation dient das Verhältnis zwischen dem Betrag der maximalen Änderung des arteriellen Drucksignals des arteriellen Drucksensors und dem Betrag der maximalen Änderung des venösen Drucksignals des venösen Drucksensors. Mit der Auswerteinheit wird die Rezirkulation Rez [%] nach der folgenden Gleichung berechnet.

Der Zusammenhang zwischen der erzielten Hämatokritänderung Δ*Hkt* und der gemessenen Druckänderung (Δpₐ und Δpᵥ) auf der venösen und arteriellen Seite ist aber nicht linear. Außerdem besteht eine Abhängigkeit vom Blutfluss Q_{b}, der Geometrie der Kanüle, insbesondere deren Durchmesser, und dem absoluten Hämatokritwert. Die erfindungsgemäße Vorrichtung sieht daher vorzugsweise eine Messung unter definierten Bedingungen vor, wobei Q_{b} und Qₛ konstant sind, so dass die Rezirkulation Rez [%] nach der obigen Gleichung mit einer hinreichenden Genauigkeit bestimmt werden kann.

Im Gegensatz zu einer Messung auf der Grundlage einer Verdickung anstelle einer Verdünnung des Bluts stellt sich nicht das Problem, dass sich der Dialysator zusetzen kann oder sich Blutgerinnsel im Schlauchsystem bilden können. Darüber hinaus zeigen sich bei dem erfindungsgemäßen Messverfahren aufgrund der Erfassung der Druckänderung unmittelbar nach der Hämodilution keine Auswirkungen von der kardiopulmonalen Rezirkulation auf das Messergebnis.

## Patentansprüche

1. Vorrichtung zur Erkennung der Rezirkulation für eine extrakorporale Blutbehandlungsvorrichtung, wobei die extrakorporale Blutbehandlungsvorrichtung aufweist:
einen extrakorporalen Blutkreislauf (5A) mit einer arteriellen Blutleitung (5), die zu der ersten Kammer (3) eines durch eine Membran (2) in die erste Kammer (3) und eine zweite Kammer (4) unterteilten Dialysators (1) oder Filters führt, und einer venösen Blutleitung (6), die von der ersten Kammer des Dialysators oder Filters abgeht,
ein Flüssigkeitssystem (5B), das die zweite Kammer (4) des Dialysators (1) oder Filters umfasst,
eine Einrichtung (8) zum Fördern von Blut mit einer vorgegebenen Blutflussrate Q_{b} durch die arterielle Blutleitung (6) in die erste Kammer des Dialysators oder Filters und durch die venöse Blutleitung (7) aus der ersten Kammer des Dialysators oder Filters,
eine Einrichtung (16) zum Zuführen von Substituat dem Blut mit einer vorgegebenen Substituatrate Qₛ in der arteriellen Blutleitung (6) stromauf des Dialysators oder Filters,
wobei die Vorrichtung zur Bestimmung der Rezirkulation aufweist:
eine Messeinheit (18) zum Messen des Drucks im extrakorporalen Kreislauf in der arteriellen und/oder venösen Blutleitung (6, 7), und eine
Auswerteinheit (19) zur Erkennung der Rezirkulation auf der Grundlage der Druckmessung,
wobei
die Vorrichtung zur Erkennung der Rezirkulation eine mit der Einrichtung (8) zum Fördern von Blut und der Einrichtung (16) zum Zuführen von Substituat zusammenwirkende Steuereinheit (17) aufweist, die derart ausgebildet ist, dass in einem Betriebsmodus zur Erkennung der Rezirkulation,
die Einrichtung (16) zum Zuführen von Substituat derart angesteuert wird, dass in einem vorgegebenen Zeitintervall Substituat mit einer vorgegebenen Substituatrate Qₛ dem Blut in der arteriellen Blutleitung stromauf des Dialysators oder Filters zugeführt wird, und
die Auswerteinheit (19) derart ausgebildet ist, dass der von der Druckmesseinheit (18) in der arteriellen und/oder venösen Blutleitung gemessene Druck zur Erkennung einer Rezirkulation auf eine Änderung des Drucks überwacht wird.
**dadurch gekennzeichnet, dass**
die Einrichtung (8) zum Fördern von Blut derart angesteuert wird, dass in dem vorgegebenen Zeitintervall die vorgegebene erste Blutflussrate Q_{b1} auf eine vorgegebene zweite Blutflussrate Q_{b2} reduziert wird.

2. Vorrichtung nach Anspruch 1, **dadurch gekennzeichnet, dass** die Auswerteinheit (19) derart ausgebildet ist, dass bei der Erkennung einer Druckerhöhung in der arteriellen Blutleitung ein das Vorliegen einer Rezirkulation signalisierendes Steuersignal erzeugt.

3. Vorrichtung nach Anspruch 1, **dadurch gekennzeichnet, dass** die Auswerteinheit (19) derart ausgebildet ist, dass bei der Erkennung einer Druckverringerung in der venösen Blutleitung ein das Vorliegen einer Rezirkulation signalisierendes Steuersignal erzeugt.

4. Vorrichtung nach einem der Ansprüche 1 bis 3, **dadurch gekennzeichnet, dass** die Vorrichtung zur Bestimmung der Rezirkulation eine das Steuersignal der Auswerteinheit (19) empfangende optische und/oder akustische Signaleinheit (21) aufweist, die derart ausgebildet ist, dass die Signaleinheit ein akustisches und/oder optisches Signal erzeugt, wenn die Signaleinheit von der Auswerteinheit das Steuersignal empfängt.

5. Vorrichtung nach einem der Ansprüche 1 bis 4, **dadurch gekennzeichnet, dass** die Auswerteinheit (19) derart ausgebildet ist, dass der Betrag der Änderung des Drucks in der arteriellen oder venösen Blutleitung mit einem vorgegebenen Grenzwert verglichen wird, wobei die Auswerteinheit (19) ein das Vorliegen einer Rezirkulation anzeigendes Steuersignal erzeugt, wenn der Betrag der Druckänderung größer als der vorgegebene Grenzwert ist.

6. Vorrichtung nach einem der Ansprüche 1 bis 5, **dadurch gekennzeichnet, dass** die Steuereinheit (17) derart ausgebildet ist, dass die Einrichtung (16) zum Zuführen von Substituat in dem vorgegebenen Zeitintervall in dem Betriebsmodus zur Erkennung der Rezirkulation derart angesteuert wird, dass die Differenz zwischen der vorgegebenen Substituatrate Qₛ und der Differenz zwischen der ersten Blutflussrate Q_{b1} und der zweiten Blutflussrate Q_{b2} einem bestimmten Wert entspricht.

7. Vorrichtung nach Anspruch 6, **dadurch gekennzeichnet, dass** der bestimmte Wert null ist.

8. Vorrichtung nach einem der Ansprüche 1 bis 7, **dadurch gekennzeichnet, dass** die Auswerteinheit (18) derart ausgebildet ist, dass das Verhältnis zwischen dem Betrag der Änderung des von der Druckmesseinheit (18) in der arteriellen Blutleitung gemessenen Drucks und dem Betrag der Änderung des in der venösen Blutleitung gemessenen Drucks berechnet wird.

9. Vorrichtung nach einem der Ansprüche 1 bis 8, **dadurch gekennzeichnet, dass** eine Betätigungseinheit (21) für die Steuereinheit (17) vorgesehen ist, die derart ausgebildet ist, dass nach Betätigung eines Betätigungsorgans (21A) die Steuereinheit den Betriebsmodus zur Erkennung der Rezirkulation vorgibt, in dem die Einrichtung (8) zum Fördern von Blut und die Einrichtung (16) zum Zuführen von Substituat angesteuert werden.

10. Extrakorporale Blutbehandlungsvorrichtung, die aufweist:
einen extrakorporalen Blutkreislauf (5A) mit einer arteriellen Blutleitung (6), die zu der ersten Kammer (3) eines durch eine Membran (2) in die erste Kammer (3) und eine zweite Kammer (4) unterteilten Dialysators (1) oder Filters führt, und einer venösen Blutleitung (7), die von der ersten Kammer des Dialysators oder Filters abgeht,
ein Flüssigkeitssystem (5B), das die zweite Kammer (4) des Dialysators (1) oder Filters umfasst,
eine Einrichtung (8) zum Fördern von Blut mit einer vorgegebenen Blutflussrate Q_{b} durch die arterielle Blutleitung in die erste Kammer des Dialysators oder Filters und durch die venöse Blutleitung aus der ersten Kammer des Dialysators oder Filters,
eine Einrichtung (16) zum Zuführen von Substituat dem Blut mit einer vorgegebenen Substituatrate Qₛ in der arteriellen Blutleitung stromauf des Dialysators oder Filters,
**dadurch gekennzeichnet, dass** die
Blutbehandlungsvorrichtung eine Vorrichtung zur Bestimmung der Rezirkulation nach einem der Ansprüche 1 bis 9 aufweist.

## Claims

1. A device for detecting the recirculation for an extracorporeal blood treatment apparatus, wherein the extracorporeal blood treatment apparatus comprises:
an extracorporeal blood circuit (5A) with an arterial blood line (5), which leads to a first chamber (3) of a dialyser (1) or filter divided by a membrane (2) into the first chamber (3) and a second chamber (4), and a venous blood line (6), which leads away from the first chamber of the dialyser or filter,
a fluid system (5B), which comprises the second chamber (4) of the dialyser (1) or filter,
a device (8) for conveying blood at a preset blood flow rate Q_{b} through the arterial blood line (6) into the first chamber of the dialyser or filter and through the venous blood line (7) from the first chamber of the dialyser or filter,
a device (16) for supplying substituate to the blood at a preset substituate rate Qₛ in the arterial blood line (6) upstream of the dialyser or filter,
wherein the device for determining the recirculation comprises:
a measuring unit (18) for measuring the pressure in the extracorporeal circuit in the arterial and/or venous blood line (6, 7), and
an evaluation unit (19) for detecting the recirculation on the basis of the pressure measurement,
the device for detecting the recirculation comprising a control unit (17) cooperating with the device (8) for conveying blood and the device (16) for supplying substituate, said control unit being constituted such that, in an operating mode for detecting the recirculation,
the device (16) for supplying substituate is controlled in such a way that in a preset time interval substituate is fed at a preset substituate rate Qₛ to the blood in the arterial blood line upstream of the dialyser or filter, and
the evaluation unit (19) being constituted such that the pressure measured by the pressure measuring unit (18) in the arterial and/or venous blood line for the detection of a recirculation is monitored for a change in the pressure
**characterised in that**
the device (8) for conveying blood is controlled in such a way that in the preset time interval the preset first blood flow rate Q_{b1} is reduced to a preset second blood flow rate Q_{b2}.

2. The device according to claim 1, **characterised in that** the evaluation unit (19) is constituted such that a control signal signaling the presence of a recirculation is generated when a pressure increase is detected in the arterial blood line.

3. The device according to claim 1, **characterised in that** the evaluation unit (19) is constituted such that the control signal signaling the presence of a recirculation is generated when a pressure reduction is detected in the venous blood line.

4. The device according to any one of claims 1 to 3, **characterised in that** the device for determining the recirculation comprises an optical and/or acoustic signal unit (21) receiving the control signal of the evaluation unit (19), said signal unit being constituted such that the signal unit generates an acoustic and/or optical signal when the signal unit receives the control signal from the evaluation unit.

5. The device according to any one of claims 1 to 4, **characterised in that** the evaluation unit (19) is constituted such that the amount of the change in the pressure in the arterial or venous blood line is compared with a preset threshold value, wherein the evaluation unit (19) generates a control signal indicating the presence of a recirculation when the amount of the pressure change is greater than the preset threshold value.

6. The device according to any one of claims 1 to 5, **characterised in that** the control unit (17) is constituted such that the device (16) for supplying substituate in the preset time interval in the operating mode for detecting the recirculation is controlled in such a way that the difference between preset substituate rate Qₛ and the difference between first blood flow rate Q_{b1} and second blood flow rate Q_{b2} corresponds to a specific value.

7. The device according to claim 6, **characterised in that** the specific value is zero.

8. The device according to any one of claims 1 to 7, **characterised in that** the evaluation unit (18) is constituted such that the ratio between the amount of the change in the pressure measured by the pressure measuring unit (18) in the arterial blood line and the amount of the change in the pressure measured in the venous blood line is calculated.

9. The device according to any one of claims 1 to 8, **characterised in that** an actuation unit (21) is provided for the control unit (17), said actuation unit-being constituted such that, after actuation of an actuation element (21A), the control unit selects the operating mode for detecting the recirculation, in which the device (8) for conveying blood and the device (16) for supplying substituate are controlled.

10. An extracorporeal blood treatment apparatus, which comprises:
an extracorporeal blood circuit (5A) with an arterial blood line (5), which leads to a first chamber (3) of a dialyser (1) or filter divided by a membrane (2) into the first chamber (3) and a second chamber (4), and a venous blood line (7), which leads away from the first chamber of the dialyser or filter,
a fluid system (5B), which comprises the second chamber (4) of the dialyser (1) or filter,
a device (8) for conveying blood at a preset blood flow rate Q_{b} through the arterial blood line into the first chamber of the dialyser or filter and through the venous blood line from the first chamber of the dialyser or filter,
a device (16) for supplying substituate to the blood at a preset substituate rate Qₛ in the arterial blood line upstream of the dialyser or filter,
**characterised in that**
the blood treatment apparatus comprises a device for determining the recirculation according to any one of claims 1 to 9.

## Revendications

1. Dispositif permettant d'identifier la recirculation pour un dispositif de traitement sanguin extracorporel, dans lequel le dispositif de traitement sanguin extracorporel présente :
un circuit sanguin extracorporel (5A) avec une ligne sanguine artérielle (5), qui conduit à la première chambre (3) d'un dialyseur (1) ou filtre subdivisé par une membrane (2) en la première chambre (3) et une deuxième chambre (4), et une ligne sanguine veineuse (6), qui part de la première chambre du dialyseur ou filtre,
un système de fluide (5B), qui comprend la deuxième chambre (4) du dialyseur (1) ou filtre,
un dispositif (8) de transport de sang avec un débit sanguin prédéfini Q_{b} à travers la ligne sanguine artérielle (6) dans la première chambre du dialyseur ou filtre et à travers la ligne sanguine veineuse (7) depuis la première chambre du dialyseur ou filtre,
un dispositif (16) d'amenée d'une solution de substitution au sang avec un débit de solution de substitution prédéfini Qₛ dans la ligne sanguine artérielle (6) en amont du dialyseur ou filtre,
dans lequel le dispositif de détermination de la recirculation présente :
une unité de mesure (18) pour mesurer la pression dans le circuit extracorporel dans la ligne sanguine artérielle et/ou veineuse (6, 7), et une
unité d'évaluation (19) pour l'identification de la recirculation sur la base de la mesure de pression,
dans lequel
le dispositif permettant d'identifier la recirculation présente une unité de commande (17) coopérant avec le dispositif (8) de transport de sang et le dispositif (16) d'amenée d'une solution de substitution, qui est réalisé de telle sorte que dans un mode de fonctionnement d'identification de la recirculation,
le dispositif (16) d'amenée d'une solution de substitution est commandé de telle sorte que dans un intervalle de temps prédéfini une solution de substitution est amenée avec un débit de solution de substitution prédéfini Qₛ au sang, dans la ligne sanguine artérielle en amont du dialyseur ou filtre, et
l'unité d'évaluation (19) est réalisée de telle sorte que la pression mesurée par l'unité de mesure de pression (18) dans la ligne sanguine artérielle et/ou veineuse pour l'identification d'une recirculation est surveillée quant à une modification de la pression,
**caractérisé en ce que**
le dispositif (8) de transport de sang est commandé de telle sorte que dans l'intervalle de temps prédéfini, le premier débit sanguin prédéfini Q_{b1} est réduit à un deuxième débit sanguin prédéfini Q_{b2}.

2. Dispositif selon la revendication 1, **caractérisé en ce que** l'unité d'évaluation (19) est réalisée de telle sorte que lors de l'identification d'une augmentation de pression dans la ligne sanguine artérielle, un signal de commande signalant la présence d'une recirculation est généré.

3. Dispositif selon la revendication 1, **caractérisé en ce que** l'unité d'évaluation (19) est réalisée de telle sorte que lors de l'identification d'une diminution de pression dans la ligne sanguine veineuse, un signal de commande signalant la présence d'une recirculation est généré.

4. Dispositif selon l'une quelconque des revendications 1 à 3, **caractérisé en ce que** le dispositif de détermination de la recirculation présente une unité de signalisation optique et/ou acoustique (21) recevant le signal de commande de l'unité d'évaluation (19), qui est réalisé de telle sorte que l'unité de signalisation génère un signal acoustique et/ou optique, lorsque l'unité de signalisation reçoit le signal de commande de l'unité d'évaluation.

5. Dispositif selon l'une quelconque des revendications 1 à 4, **caractérisé en ce que** l'unité d'évaluation (19) est réalisée de telle sorte que la valeur de la modification de la pression dans la ligne sanguine artérielle ou veineuse est comparée avec une valeur limite prédéfinie, dans lequel l'unité d'évaluation (19) génère un signal de commande indiquant la présence d'une recirculation, lorsque la valeur de la modification de pression est supérieure à la valeur limite prédéfinie.

6. Dispositif selon l'une quelconque des revendications 1 à 5, **caractérisé en ce que** l'unité de commande (17) est réalisée de telle sorte que le dispositif (16) d'amenée d'une solution de substitution est commandé dans l'intervalle de temps prédéfini dans le mode de fonctionnement d'identification de la recirculation de telle sorte que la différence entre le débit de solution de substitution prédéfini Qₛ et la différence entre le premier débit sanguin Q_{b1} et le deuxième débit sanguin Q_{b2} correspond à une valeur déterminée.

7. Dispositif selon la revendication 6, **caractérisé en ce que** la valeur déterminée est zéro.

8. Dispositif selon l'une quelconque des revendications 1 à 7, **caractérisé en ce que** l'unité d'évaluation (18) est réalisée de telle sorte que le rapport entre la valeur de la modification de la pression mesurée dans la ligne sanguine artérielle par l'unité de mesure de pression (18) et la valeur de la modification de la pression mesurée dans la ligne sanguine veineuse est calculé.

9. Dispositif selon l'une quelconque des revendications 1 à 8, **caractérisé en ce qu'**une unité d'actionnement (21) est prévue pour l'unité de commande (17), qui est réalisée de telle sorte qu'après actionnement d'un organe d'actionnement (21A), l'unité de commande prédéfinit le mode de fonctionnement d'identification de la recirculation, par le fait que le dispositif (8) de transport de sang et le dispositif (16) d'amenée d'une solution de substitution sont commandés.

10. Dispositif de traitement sanguin extracorporel, qui présente :
un circuit sanguin extracorporel (5A) avec une ligne sanguine artérielle (6), qui conduit à la première chambre (3) d'un dialyseur (1) ou filtre subdivisé par une membrane (2) en la première chambre (3) et une deuxième chambre (4), et une ligne sanguine veineuse (7), qui part de la première chambre du dialyseur ou filtre,
un système de fluide (5B), qui comprend la deuxième chambre (4) du dialyseur (1) ou filtre,
un dispositif (8) de transport de sang avec un débit sanguin prédéfini Q_{b} à travers la ligne sanguine artérielle dans la première chambre du dialyseur ou filtre et à travers la ligne sanguine veineuse depuis la première chambre du dialyseur ou filtre,
un dispositif (16) d'amenée d'une solution de substitution au sang avec un débit de solution de substitution prédéfini Qₛ dans la ligne sanguine artérielle en amont du dialyseur ou filtre,
**caractérisé en ce que** le
dispositif de traitement sanguin présente un dispositif de détermination de la recirculation selon l'une quelconque des revendications 1 à 9.
